# EUROPEAN PATENT SPECIFICATION

(11) **EP 3 357 442 B1**
(45) Date of publication and mention of the grant of the patent: **17.12.2025**
(21) Application number: 16850131.0
(22) Date of filing: 08.06.2016
(51) Int. Cl.: A61B 17/70, A61B 17/88, A61B 17/86

(54) **TRANSVERSE SHIFTING SCREW-TAIL, LATERALLY ADJUSTABLE SPINAL SCREW**
SCHRAUBENENDE MIT TRANSVERSALER VERSCHIEBUNG, SEITLICH VERSTELLBARE PEDIKELSCHRAUBE
QUEUE DE VIS À DÉCALAGE TRANSVERSAL, VIS VERTÉBRALE RÉGLABLE LATÉRALEMENT

(30) Priority: 30.09.2015 CN 201510642652; 30.09.2015 CN 201520771288 U
(43) Date of publication of application: 08.08.2018
(73) Proprietor: Shanghai Sanyou Medical Co., Ltd., Jiading District, Shanghai 201807 (CN)
(72) Inventor: LIU, Michael Mingyan, Shanghai 201807 (CN); QIU, Yong, Shanghai 201807 (CN); SONG, Yueming, Shanghai 201807 (CN); CHEN, Qixin, Shanghai 201807 (CN); LEHUEC, Jean-Charles, Shanghai 201807 (CN); LIU, Ruifeng, Shanghai 201807 (CN)
(74) Representative: Petraz, Gilberto Luigi
(86) International application number: PCT/CN2016/085194
(87) International publication number: WO 2017/054506

(56) References cited:
- EP-A1- 2 221 012
- CN-A- 102 596 066
- CN-A- 103 565 502
- CN-U- 202 161 394
- CN-U- 203 790 021
- CN-U- 205 107 855
- CN-Y- 201 143 228
- US-A- 5 545 228
- US-A1- 2003 171 755
- US-A1- 2004 054 371
- US-A1- 2006 247 631
- US-A1- 2010 160 977
- US-A1- 2010 198 272
- US-A1- 2012 221 055
- US-B1- 6 328 739

## Description

### Background of the Present Invention

### Field of Invention

The present invention further relates to a transverse adjustable spinal screw.

### Description of Related Arts

Spinal internal fixation technique was started to be applied since 1960s for treating various spinal diseases such as spine malformation, adolescent scoliosis, spinal fracture and spinal degeneration through operations. Spinal internal fixation technique may be briefly described as the following several steps: firstly, selecting a spinal segment which needs to be fixed, and implanting anchoring appliances (such as hooks or screws) into specific positions on all or partial vertebral bodies at two ends or in this segment; then locking connecting appliances (such as fixing steel plate or fixing rods) to each hook or screw to restrict relatively motion within the spinal segment such that the spinal segment is enabled to become a stable body; and finally, placing bone graft materials (such as autogenous bones or bone substitute materials) between vertebral bodies in this segment to expect that the spinal segment is gradually incorporated to form an integral body within a period of time after an operation, so as to guarantee long-term stability.

Each spine can be divided into an anterior portion and a posterior portion, the anterior portion is the portion towards the direction of abdomen, a posterior portion is the portion towards the direction of back, and the bone tissues connecting the anterior portion and the posterior portion are called as vertebral pedicles. All screws which enter bones from the posterior portion and are implanted into vertebral bodies through vertebral pedicles are collectively called as vertebral pedicle screws, and usually screw seat left at the posterior portion are provided with rod grooves for locking a connecting rod.

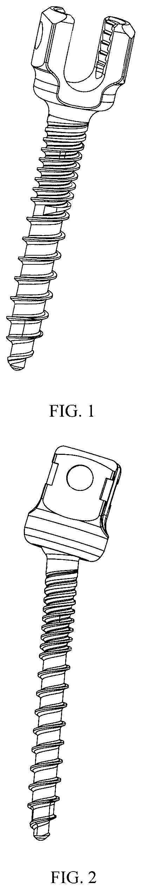

Vertebral pedicle screws are anchoring appliances which are the most commonly used at present in a spinal internal fixation system, and each vertebral pedicle screw consists of a screw tail (threaded portion implanted into a bone) and a screw seat (other portion left outside the bone). In general, vertebral pedicle screws are divided into fixed-angle screws (as illustrated in FIG. 1, wherein the screw tail and the screw seat are integrated) and variable-angle screws (as illustrated in FIG. 2, wherein the screw tail and the screw seat have a certain folding adjustable angle).

Usually a plurality of screws are used in one spinal fixation operation, the screws are respectively implanted into each vertebral body which needs to be fixed, and then a connecting rod is installed into each screw grooves and is locked to finally achieve the purpose of fixing a spine. Implantation of screws mainly considers anatomical characteristics of target vertebral bodies and restriction of an operation space, and thus positions and directions from which screws enter bones are possibly very different even though the screws are located between two adjacent vertebral bodies. Fixed-angle screws have the disadvantages that the angle on the lateral plan of spine cannot be adjusted, and vertebral bodies might be excessively bent or extended when the connecting rod is locked. While, by using adjustable-angle screws, this kind of problems can be avoided to a very great extent. However, on the front plan of a spine, screws implanted into different vertebral bodies are possibly dislocated in a left-right direction, and consequently it will cause a very great difficulty to installation of the connecting rod, especially when a plurality of screws are used. Usually, a doctor has to make some compromises such as linearly arranging screws, which will result in difficult to guarantee that each screw is implanted under an ideal state, or cooperatively connecting each screw seat by bending the connecting rod, which will make the operation difficulty and complex, and increase a certain uncertainty or cause improperly distorting of a spine.

Up to now, there have been no spinal internal fixation appliances and methods which can satisfactorily solve this problem.

Spine fixation systems known in the art are disclosed, by way of example, in patent documents US 2012/221055 and US 2003/171755 that describes systems with very complicated structure that are costly and not easy to use since they make the procedures more complicated during the surgical operation.

US20100160977 A1 discloses a coupling device for securing an elongate member to the spine. The coupling device comprises a compressible inner member that secures an anchor member therein when the inner member is axially shifted within an outer member. The elongate member is retained within the device by an axially inserted locking member, and may be secured independently of the anchor member. The coupling device and anchor may be configured to provide increased angulation of the anchor with respect to the coupling device. For instance, anchor member may have an offset head portion in order to provide normal pivoting of the coupling device when the anchor is attached to bone at an angle.

### Summary of the Present Invention

The present invention relates a transverse adjustable spinal screw as defined in claim

### 1. One preferred embodiment is set forth in the dependent claim.

In view of the above-mentioned disadvantages of the prior art, the purpose of the present invention is to provide a transverse adjustable spinal screw, which are used for solving the problem that it is difficult to linearly arrange a plurality of screws implanted into vertebral bodies in the prior art.

The transverse shift screw tail and the transverse adjustable spinal screw provided by the present invention have the following beneficial effects:
According to the transverse shift screw tail and the transverse adjustable spinal screw, by designing a brand new screw, the screw seats are connected linearly, the installation process of the connecting rod is enabled to be reliable and effective, a spine is prevented from being improperly distorted, the difficulty of operation by a doctor is reduced and the operation effect is improved.

### Brief Description of the Drawings

FIG. 1 illustrates a structural schematic view of a fixed-angle screw in the prior art.
FIG. 2 illustrates a structural schematic view of a variable-angle screw in the prior art.
FIG. 3 illustrates a structural schematic view of a transverse shift screw tail in the present invention.
FIG. 4 illustrates a front view of the transverse shift screw tail illustrated in FIG. 3.
FIG. 5 illustrates a structural schematic view of a transverse adjustable spinal screw in the present invention.
FIG. 6 illustrates a structural schematic view of a screw seat of the transverse adjustable spinal screw illustrated in FIG. 5.
FIG. 7 illustrates a stereoscopic schematic view of the screw seat illustrated in FIG. 6.
FIG. 8 illustrates a structural schematic view of a slidable tensioning ring illustrated in FIG. 6.
FIGs. 9-14 illustrate schematic views of steps of a method for implanting the transverse adjustable spinal screw illustrated in FIG. 5.

### Description of component mark numbers

- 1: Screw tail
- 11: Fixing column
- 12: Platform
- 13: Installation head
- 14: Installation adjusting hole
- 2: Screw seat
- 21: Rod groove
- 22: Locking thread
- 23: Screw seat sleeve
- 24: Slidable tensioning ring
- 25: Ball socket
- 26: Opening
- 27: Extension groove
- 28: Limiting ring
- 29: Loose section
- 210: Limiting groove
- 211: Limiting screw
- 212: Arc groove
- 3: Connecting rod
- 4: Locking bolt
- 5: Special screwdriver for holding screw tail
- 6: Vertebral body

### Detailed Descriptions of the Preferred Embodiments

The implementation modes of the present invention will be described below through specific embodiments. One skilled in the art can easily understand other advantages and effects of the present invention according to contents disclosed by the description.

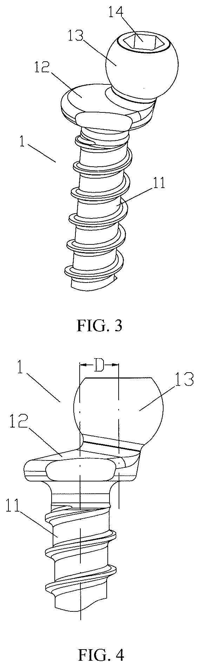

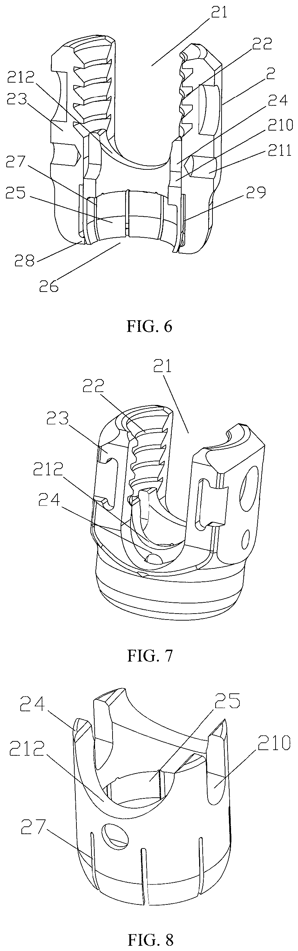

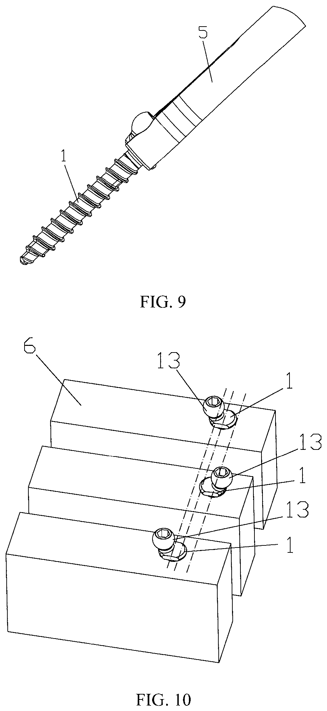

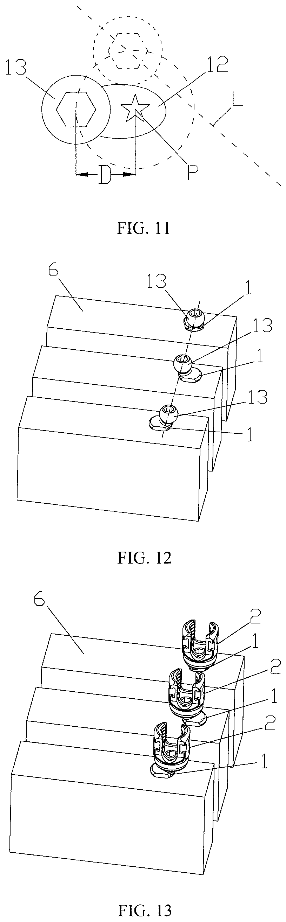

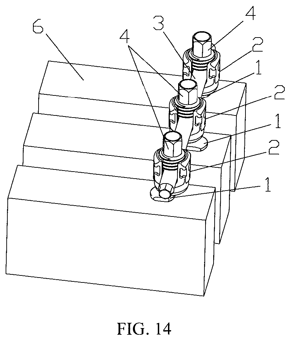

Please refer to FIG. 3 to FIG. 14. It shall be noted that the structures, scales, sizes and the like illustrated in the drawings of the description are only used for cooperating with the contents disclosed by the description to allow one skilled in the art to understand and read instead of limiting the implementable limitation conditions of the present invention, and thus have no technical substantive meanings; and any structural modifications, changes of scaling relations or adjustments to sizes shall still fall into the scope which can be covered by the technical contents disclosed by the present invention under the situation that the effects which can be produced by the present invention and the purposes which can be achieved by the present invention are not influenced. In addition, direction or quantification words such as "above", "below", "left", "right", "middle" and "one" cited in the description are just used for facilitating clear description instead of limiting the implementable scope of the present invention.

As illustrated in FIG. 3 to FIG. 4, the present invention provides a transverse shift screw tail 1, the screw tail 1 comprises a fixing column 11 capable of being screwed into a vertebral body 6 and a platform 12 located at an end portion of the fixing column 11, the platform 12 is provided with an installation head 13 used for connecting with a screw seat 2, and an eccentric distance is provided between a center of the installation head 13 and an axis of the fixing column 11.

By adopting the eccentric installation head 13, the position of the installation head 13 can be adjusted by rotating the fixing column 11, such that the center of the installation head 13 is enabled to approach the reference line L (i.e., the placement position of the connecting rod 3) to the utmost extent, thus the screw seat 2 installed on the installation head 13 can be enabled to be located on the same line as much as possible, all screw seats 2 can be connected through one connecting rod 3, the spinal screws are located at the optimal positions on the spine and the spine is not caused to be improperly distorted.

A range of the eccentric distance D between the center of the installation head 13 and the axis of the fixing column 11 is 1mm-5mm, and the range of the eccentric distance D substantially satisfies a range that the screw tail 1 rotates for 1/4 circle, such that the installation head 13 can approach the requirement of reference line L to the utmost extent.

In order to facilitate the fixation of the screw tail 1 into the vertebral body 6, an outer circumferential surface of the fixing column 11 is provided with a thread.

An outer surface of the installation head 13 is preferably a spherical surface and a top of the installation head 13 is provided with an inwards recessed noncircular installation adjusting hole 14. A cross section of the installation adjusting hole 14 is preferably hexagonal, and thus a hexagonal screwdriver can be inserted into the installation adjusting hole 14 and used for adjusting the position of the installation head 13.

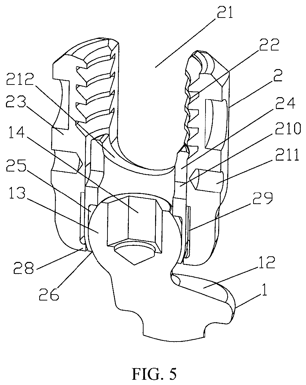

As illustrated in FIG. 5 to FIG. 8, the present invention provides a transverse adjustable spinal screw, comprising the above-mentioned screw tail 1 and a screw seat 2 installed on an installation head 13 of the screw tail 1, a top of the screw seat 2 is provided with a rod groove 21 used for placing a connecting rod 3, the rod groove 21 is recessed downwards from the top of the screw seat 2 and radially penetrates the screw seat 2, a bottom of the rod groove 21 is a curved surface adapted to an outer circumferential surface of the connecting rod 3, and a side surface of the rod groove 21 is provided with a locking thread 22 fit with a locking bolt 4.

Since the screw seat 2 has a certain transverse shift relative to the fixing column 11 of the screw tail 1, if the screw seat 2 is installed on the screw tail 1 in advance, when the screw tail 1 is rotated, the movement periphery of the screw seat 2 is larger and the operation is not facilitated. Therefore, the present invention provides a design of a screw seat, assembly that after the screw tail 1 is fixed on the vertebral body 6, the screw seat 2 is installed on the screw tail 1. The screw seat 2 installed on the installation head 13 is capable of rotating on the installation head 13 such that axis of the rod grooves 21 of a plurality of screw seats 2 form a line, and the connecting rod 3 can be placed in the rod grooves 21 to connect the plurality of rod grooves 21 together. In order to fix the connecting rod 3, a locking bolt 4 is installed at the top of the rod groove 21 and the locking bolt is fit with the locking thread 22 on the side surface of the rod groove 21.

One specific structure of the screw seat 2 is as follow: the screw seat 2 comprises a screw seat sleeve 23 and a slidable tensioning ring 24 which is sleeve-mounted into the screw seat sleeve 23, the slidable tensioning ring 24 is sleeve-mounted onto the installation head 13 of the screw tail 1, and the rod groove 21 is located at one end, far away from the slidable tensioning ring 24, of the screw seat sleeve 23.

The slidable tensioning ring 24 is provided with a ball socket 25 for placing the installation head 13, the ball socket 25 is provided with an opening 26 facing to the installation head 13, and a diameter of the opening 26 is smaller than a maximum diameter of the installation head 13. The installation head 13 can rotate at a plurality of angles in the ball socket 25 such that the screw seat 2 can be adjusted at a plurality of angles.

A circumferential surface of the ball socket 25 is provided with a plurality of extension grooves 27 which are uniformly distributed along a circumferential direction, and the extension grooves 27 extend from the opening 26 to one end corresponding to the opening 26. The ball socket 25 which is provided with the extension grooves is petal-shaped. In the process that the installation head 13 is placed into the ball socket 25 through the opening 26, by arranging the arrangement of the extension groove 27, enables the ball socket 25 to have certain elasticity and can be slightly outwards extended to guarantee smooth installation of the installation head 13; and when the connecting rod 3 is installed into the rod grooves 21, the connecting rod 3 will apply certain pressure to the slidable tensioning ring 24, the arrangement of the extension grooves 27 enables the ball socket 25 to have certain elasticity, and the length of the slidable tensioning ring 24 in an axial direction be properly changed to adapt to the installation of the connecting rod 3.

In order to prevent the slidable tensioning ring 24 from falling out of the screw seat sleeve 23, one end, facing to the screw tail 1, of an inner circumferential surface of the screw seat sleeve 23 is provided with a limiting ring 28 and an inner circumferential surface of the limiting ring 28 is fit with an outer circumferential surface of the opening 26 of the ball socket 25.

A portion, corresponding to the ball socket 25, of the inner circumferential surface of the screw seat sleeve 23 is provided with an inwards recessed loose section 29 and a gap is kept between the loose section 29 and an outer circumferential surface of the ball socket 25, so as to provide a space for deformation of the ball socket 25.

A side wall of the slidable tensioning ring 24 is further provided with at least one limiting groove 210 which extends downwards from a top, and the screw seat sleeve 23 is provided with a limiting screw 211 corresponding to the limiting groove 210. When the slidable tensioning ring 24 is inserted into a predetermined position in the screw seat sleeve 23, the limiting screw 211 is clamped at the bottom of the limiting groove 210 to lock relative positions of the slidable tensioning ring 24 and the screw seat sleeve 23.

One end, facing to the rod groove 21, of the slidable tensioning ring 24 is provided with an arc groove 212, an inner circumferential surface of the arc groove 212 corresponds to the bottom of the rod groove 21, and when the slidable tensioning ring 24 is installed in place, the inner circumferential surface of the arc groove 212 protrudes out of the bottom of the rod groove 21. After the connecting rod 3 is installed into the rod groove 21, the locking bolt 4 is installed from the top of the rod groove 21. Since the inner circumferential surface of the arc groove 212 protrudes out of the bottom of the rod groove 21, the connecting rod 3 will apply certain pressure to the arc groove 212 to enable the slidable tensioning ring 24 to move downwards, and at this moment, the ball socket 25 is deformed and then locks the installation head 13, so that the overall structure of the transverse adjustable spinal screw is more stable.

To sum up, according to the transverse shift screw tail and the transverse adjustable spinal screw provided by the present invention, by designing a brand new screw, the screw seats are connected linearly, the installation process of the connecting rod is enabled to be reliable and effective, the spine is prevented from being improperly distorted, the difficulty of operation by the doctor is reduced and the operation effect is improved. Therefore, the present invention effectively overcomes various disadvantages of the prior art and thus has a great industrial utilization value.

The above-mentioned embodiments are just used for exemplarily describing the principle and effect of the present invention instead of limiting the present invention.

## Claims

1. A transverse adjustable spinal screw, comprising a screw tail (1) and a screw seat (2) installed on an installation head (13) of the screw tail (1),
wherein the screw tail (1) comprises a fixing column (11) capable of being screwed into a vertebral body (6) and a platform (12) located at an end portion of the fixing column (11), the platform (12) is provided with an installation head (13) used for connecting with a screw seat (2), an eccentric distance is provided between a center of the installation head (13) and an axis of the fixing column (11), wherein the screw tail (1), the platform (12), and the installation head (13) are integrally formed, an eccentric distance between the center of the installation head (13) and the axis of the fixing column (11) ranges 1mm-5mm;
a top of the screw seat (2) is provided with a rod groove (21) used for placing a connecting rod (3), the rod groove (21) is recessed downwards from the top of the screw seat (2) and radially penetrates the screw seat (2), a bottom of the rod groove (21) is a curved surface adapted to an outer circumferential surface of the connecting rod (3), and a side surface of the rod groove (21) is provided with a locking thread (22) fit with a locking bolt (4);
the screw seat (2) comprises a screw seat sleeve (23) and a slidable tensioning ring (24) which is sleeve-mounted into the screw seat sleeve (23), the slidable tensioning ring (24) is sleeve-mounted onto the installation head (13) of the screw tail (1), the rod groove (21) is located at one end, far away from the slidable tensioning ring (24), of the screw seat sleeve (23);
the slidable tensioning ring (24) is provided with a ball socket (25) for placing the installation head (13), the ball socket (25) is provided with an opening (26) facing to the installation head (13), and wherein a diameter of the opening (26) is smaller than a maximum diameter of the installation head (13);
wherein a portion, corresponding to the ball socket (25), of the inner circumferential surface of the screw seat sleeve (23) is provided with an inwards recessed loose section (29); wherein a circumferential surface of the ball socket (25) is provided with a plurality of extension grooves (27) which are uniformly distributed along a circumferential direction, and the extension grooves (27) extend from the opening (26) to an end corresponding to the opening (26),
wherein one end, facing to the rod groove (21), of the slidable tensioning ring (24) is provided with an arc groove (212) having an arc shape, an inner circumferential surface of the arc groove (212) corresponds to the bottom of the rod groove (21), and when the slidable tensioning ring (24) is installed in place, the inner circumferential surface of the arc groove (212) is configured to protrude out of the bottom of the rod groove (21), such that after the connecting rod (3) is installed into the rod groove (21), the locking bolt (4) is installed from the top of the rod groove (21), since the inner circumferential surface of the arc groove (212) protrudes out of the bottom of the rod groove (21), the connecting rod (3) will apply certain pressure to the arc groove (212) to enable the slidable tensioning ring (24) to move downwards so that the ball socket (25) is deformed and locks the installation head (13), a gap is kept between the loose section (29) and an outer circumferential surface of the ball socket (25) to provide a space for deformation of the ball socket (25);
wherein when the connecting rod (3) is installed into the rod groove (21), the connecting rod (3) is configured to apply certain pressure to the slidable tensioning ring (24), the plurality of extension grooves (27) is configured to enable the ball socket (25) to have certain elasticity, and a length of the slidable tensioning ring (24) in an axial direction is configured to be properly changed to adapt to installation of the connecting rod (3); **characterized in that** a sidewall of the slidable tensioning ring (24) is further provided with at least one limiting groove (210) which extends downwards from a top, and the screw seat sleeve (23) is provided with a limiting screw (211) corresponding to the limiting groove (210).

2. The transverse adjustable spinal screw according to claim 1, **characterized in that** one end, facing to the screw tail (1), of an inner circumferential surface of the screw seat sleeve (23) is provided with a limiting ring (28) and an inner circumferential surface of the limiting ring (28) is fit with an outer circumferential surface of the opening (26) of the ball socket (25).

## Patentansprüche

1. Transversale verstellbare Pedikelschraube, umfassend einen Schraubenschwanz (1) und einen Schraubensitz (2), der an einem Installationskopf (13) des Schraubenschwanzes (1) installiert ist,
worin der Schraubenschwanz (1) einen Fixierstab (11), der in einen Wirbelkörper (6) eingeschraubt werden kann, und eine Plattform (12), die an einem Endabschnitt des Fixierstabs (11) gelegen ist, umfasst, die Plattform (12) mit einem Installationskopf (13) versehen ist, der benutzt wird, um sich mit einem Schraubensitz (2) zu verbinden, ein exzentrischer Abstand zwischen einer Mitte des Installationskopfes (13) und einer Achse des Fixierstabs (11) vorgesehen ist, worin der Schraubenschwanz (1), die Plattform (12) und der Installationskopf (13) einstückig ausgebildet sind, ein exzentrischer Abstand zwischen der Mitte des Installationskopfes (13) und der Achse des Fixierstabs (11) im Bereich von 1 mm - 5 mm liegt;
ein Oberteil des Schraubensitzes (2) mit einer Stangennut (21) versehen ist, die zur Platzierung einer Verbindungsstange (3) benutzt wird, die Stangennut (21) von dem Oberteil des Schraubensitzes (2) nach unten vertieft ist und den Schraubensitz (2) radial durchdringt, ein Unterteil der Stangennut (21) eine gekrümmte Fläche ist, die einer äußeren Umfangsfläche der Verbindungsstange (3) angepasst ist, und eine Seitenfläche der Stangennut (21) mit einem verriegelnden Gewinde (22) versehen ist, das zu einem Verriegelungsbolzen (4) passt;
der Schraubensitz (2) eine Schraubensitzhülse (23) und einen verschiebbaren Spannring (24), der in die Schraubensitzhülse (23) hülsenartig montiert ist, umfasst, der verschiebbare Spannring (24) auf den Installationskopf (13) des Schraubenschwanzes (1) hülsenartig montiert ist, die Stangennut (21) an einem Ende, weit weg vom verschiebbaren Spannring (24), der Schraubensitzhülse (23) gelegen ist;
der verschiebbare Spannring (24) mit einer Kugelpfanne (25) zur Platzierung des Installationskopfes (13) versehen ist, die Kugelpfanne (25) mit einer Öffnung (26) versehen ist, die dem Installationskopf (13) zugewandt ist, und worin ein Durchmesser der Öffnung (26) kleiner ist als ein Höchstdurchmesser des Installationskopfes (13);
worin ein Abschnitt, der der Kugelpfanne (25) entspricht, der inneren Umfangsfläche der Schraubensitzhülse (23) mit einem nach innen vertieften, lockeren Abschnitt (29) versehen ist; worin eine Umfangsfläche der Kugelpfanne (25) mit einer Vielzahl von Erweiterungsnuten (27) versehen ist, die entlang einer Umfangsrichtung gleichmäßig verteilt sind, und die Erweiterungsnuten (27) sich von der Öffnung (26) zu einem Ende erstrecken, das der Öffnung (26) entspricht,
worin ein Ende, das der Stangennut (21) zugewandt ist, des verschiebbaren Spannringes (24) mit einer Bogennut (212) versehen ist, die bogenförmig ist, eine innere Umfangsfläche der Bogennut (212) dem Unterteil der Stangennut (21) entspricht, und wenn der verschiebbare Spannring (24) am Platz installiert ist, die innere Umfangsfläche der Bogennut (212) dazu eingerichtet ist, aus dem Unterteil der Stangennut (21) herauszuragen, so dass, nachdem die Verbindungsstange (3) in die Stangennut (21) installiert ist, der Verriegelungsbolzen (4) von dem Oberteil der Stangennut (21) installiert ist, da die innere Umfangsfläche der Bogennut (212) aus dem Unterteil der Stangennut (21) herausragt, die Verbindungsstange (3) einen gewissen Druck auf die Bogennut (212) aufbringen wird, um dem verschiebbaren Spannring (24) zu ermöglichen, sich nach unten zu bewegen, so dass die Kugelpfanne (25) verformt ist und den Installationskopf (13) verriegelt, ein Spalt zwischen dem lockeren Abschnitt (29) und einer äußeren Umfangsfläche der Kugelpfanne (25) gehalten wird, um einen Raum für die Verformung der Kugelpfanne (25) bereitzustellen;
worin, wenn die Verbindungsstange (3) in die Stangennut (21) installiert ist, die Verbindungsstange (3) dazu eingerichtet ist, einen gewissen Druck auf den verschiebbaren Spannring (24) aufzubringen, die Vielzahl von Erweiterungsnuten (27) dazu eingerichtet ist, der Kugelpfanne (25) zu ermöglichen, eine gewisse Elastizität zu haben, und eine Länge des verschiebbaren Spannringes (24) in einer axialen Richtung dazu eingerichtet ist, ordnungsgemäß geändert zu werden, um sich der Installation der Verbindungsstange (3) anzupassen;
**dadurch gekennzeichnet, dass** eine Seitenwand des verschiebbaren Spannringes (24) ferner mit zumindest einer Begrenzungsnut (210) versehen ist, die sich nach unten von einem Oberteil erstreckt, und die Schraubensitzhülse (23) mit einer Begrenzungsschraube (211) versehen ist, die der Begrenzungsnut (210) entspricht.

2. Transversale verstellbare Pedikelschraube nach Anspruch 1, **dadurch gekennzeichnet, dass** ein Ende, das dem Schraubenschwanz (1) zugewandt ist, einer inneren Umfangsfläche der Schraubensitzhülse (23) mit einem Begrenzungsring (28) versehen ist und eine innere Umfangsfläche des Begrenzungsringes (28) zu einer äußeren Umfangsfläche der Öffnung (26) der Kugelpfanne (25) passt.

## Revendications

1. Vis vertébrale transversale réglable, comprenant une queue de vis (1) et un siège de vis (2) installé sur une tête d'installation (13) de la queue de vis (1), dans laquelle la queue de vis (1) comprend une colonne de fixation (11) pouvant être vissée dans un corps vertébral (6) et une plate-forme (12) située à une partie d'extrémité de la colonne de fixation (11), la plate-forme (12) est pourvue d'une tête d'installation (13) utilisée pour se connecter à un siège de vis (2), une distance excentrique est prévue entre un centre de la tête d'installation (13) et un axe de la colonne de fixation (11), dans laquelle la queue de vis (1), la plate-forme (12) et la tête d'installation (13) sont formées d'un seul tenant, une distance excentrique entre le centre de la tête d'installation (13) et l'axe de la colonne de fixation (11) est comprise entre 1 mm et 5 mm ;
un sommet du siège de vis (2) est pourvu d'une rainure de tige (21) utilisée pour placer une bielle (3), la rainure de tige (21) est en retrait vers le bas à partir du sommet du siège de vis (2) et pénètre radialement dans le siège de vis (2), un fond de la rainure de tige (21) est une surface incurvée adaptée à une surface circonférentielle extérieure de la bielle (3), et une surface latérale de la rainure de tige (21) est pourvue d'un filetage de verrouillage (22) adapté à un boulon de verrouillage (4) ;
le siège de vis (2) comprend un manchon de siège de vis (23) et une bague de tension coulissante (24) qui est montée en manchon dans le manchon de siège de vis (23), la bague de tension coulissante (24) est montée en manchon sur la tête d'installation (13) de la queue de vis (1), la rainure de tige (21) est située au niveau d'une extrémité, éloignée de la bague de tension coulissante (24), du manchon de siège de vis (23) ;
la bague de tension coulissante (24) est dotée d'un logement de rotule (25) pour placer la tête d'installation (13), le logement de rotule (25) est doté d'une ouverture (26) faisant face à la tête d'installation (13), et dans lequel un diamètre de l'ouverture (26) est inférieur à un diamètre maximal de la tête d'installation (13) ;
dans laquelle une partie, correspondant au logement de rotule (25), de la surface circonférentielle intérieure du manchon de siège à vis (23) est pourvue d'une section libre en retrait vers l'intérieur (29) ; dans lequel une surface circonférentielle du logement de rotule (25) est pourvue d'une pluralité de rainures d'extension (27) qui sont uniformément réparties le long d'une direction circonférentielle, et les rainures d'extension (27) s'étendent de l'ouverture (26) à une extrémité correspondant à l'ouverture (26),
dans laquelle une extrémité, faisant face à la rainure de tige (21), de la bague de tension coulissante (24) est pourvue d'une rainure en arc (212) ayant une forme d'arc, une surface circonférentielle intérieure de la rainure en arc (212) correspond au fond de la rainure de tige (21), et lorsque la bague de tension coulissante (24) est installée en place, la surface circonférentielle intérieure de la rainure en arc (212) est configurée pour faire saillie hors du fond de la rainure de tige (21), de sorte qu'après que la bielle (3) soit installée dans la rainure de tige (21), le boulon de verrouillage (4) est installé par le haut de la rainure de tige (21), puisque la surface circonférentielle intérieure de la rainure en arc (212) fait saillie hors du fond de la rainure de tige (21), la bielle (3) appliquera une certaine pression sur la rainure en arc (212) pour permettre à la bague de tension coulissante (24) de se déplacer vers le bas de sorte que le logement de rotule (25) soit déformé et verrouille la tête d'installation (13), un espace est maintenu entre la section lâche (29) et une surface circonférentielle extérieure du logement de rotule (25) pour fournir un espace pour la déformation du logement de rotule (25) ;
dans laquelle lorsque la bielle (3) est installée dans la rainure de tige (21), la bielle (3) est configurée pour appliquer une certaine pression à la bague de tension coulissante (24), la pluralité de rainures d'extension (27) est configurée pour permettre au logement de rotule (25) d'avoir une certaine élasticité, et une longueur de la bague de tension coulissante (24) dans une direction axiale est configurée pour être correctement modifiée pour s'adapter à l'installation de la bielle (3) ;
**caractérisée en ce qu'**une paroi latérale de la bague de tension coulissante (24) est en outre pourvue d'au moins une rainure de limitation (210) qui s'étend vers le bas à partir d'un sommet, et le manchon de siège de vis (23) est pourvu d'une vis de limitation (211) correspondant à la rainure de limitation (210).

2. Vis vertébrale transversale réglable selon la revendication 1, **caractérisée en ce qu'**une extrémité, faisant face à la queue de vis (1), d'une surface circonférentielle intérieure du manchon de siège de vis (23) est munie d'une bague de limitation (28) et une surface circonférentielle intérieure de la bague de limitation (28) est ajustée avec une surface circonférentielle extérieure de l'ouverture (26) du logement de rotule (25).
